# EUROPEAN PATENT APPLICATION

(11) **EP 4 033 237 A1**
(43) Date of publication of application: **27.07.2022**
(21) Application number: 20865012.7
(22) Date of filing: 17.09.2020
(51) Int. Cl.: G01N 27/62, G01N 33/50, G01N 33/53, G01N 33/74, G01N 30/72, G01N 30/88

(54) **METHOD FOR ANALYZING STEROID HORMONES CONTAINED IN ANIMAL HAIR SAMPLE, STRESS EVALUATION METHOD AND METHOD FOR ANALYZING CAUSE OF HAIR LOSS**

(30) Priority: 20.09.2019 JP 2019172111
(71) Applicant: Aska Pharma Medical Co., Ltd, Kawasaki-shi, Kanagawa 215-8555 (JP)
(72) Inventor: HOBOU, Yoshitaka, Kanagawa 251-8555 (JP); MIYASHIRO, Yoshimichi, Kanagawa 251-8555 (JP); FUJIKATA, Akira, Kanagawa 251-8555 (JP)
(74) Representative: Bardehle Pagenberg Partnerschaft mbB Patentanwälte Rechtsanwälte
(86) International application number: PCT/JP2020/035191
(87) International publication number: WO 2021/054386

(57) **Abstract**

Provided is, for example, a method for analyzing steroid hormones contained in a body hair sample from an animal, wherein body hair collected from an animal is used as the body hair sample, and the method includes: an extraction step of extracting a plurality of types of steroid hormones from the body hair sample by using a 45 vol% to 55 vol% aqueous acetonitrile solution containing 0.1 M trifluoroacetic acid; and a measurement step of measuring the amounts of the plurality of types of steroid hormones extracted.

## Description

### Technical Field

The present invention relates to a method for analyzing steroid hormones contained in an animal body hair sample, a method for stress evaluation, and a method for analyzing a cause of hair loss.

### Cross Reference to Related Applications

The present application is based on Japanese Patent Application No. 2019-172111 filed on September 20, 2019, and claims priority thereto, and the entire contents of the patent application are incorporated herein by reference.

### Background Art

Steroid hormones, even in small amounts, exhibit important, wide-ranging physiological effects in vivo. Accordingly, analysis of the amounts of various steroid hormones in vivo can be utilized for evaluation and monitoring not only of diseases, but also of health condition in a broad sense, determination of drug usage, and so on (Patent Literature 1). For example, cortisone, cortisol, corticosterone, dehydroepiandrosterone (DHEA), and the like are known to serve as indicators of stress, and dihydrotestosterone (DHT), testosterone and the like are known to serve as indicators of androgenetic alopecia (AGA). Testosterone and dehydroepiandrosterone are known to have anabolic effect, and each used even for antiaging as a rejuvenating hormone or the like, and androstenedione, androstenediol, and the like are expected to exhibit the same effect.

In general, body hair can be collected without significant invasion, and hence analysis of steroid hormones by using animal body hair as a sample has become a common practice in recent years.

Methanol has been commonly used as a solvent to extract chemicals from the body hair of animals (Non Patent Literature 1); however, use of methanol as an extraction solvent for steroid hormones requires long period of time for extraction processes, and, moreover, had the problem of low extraction efficiency.

For these reasons, 10 to 100 mg of a body hair sample is needed for analyzing a plurality of steroid hormones from a single sample by using conventional analysis methods; in the case of human hair, for example, tens to hundreds of strands of hair have to be collected (Non Patent Literatures 1 and 2), which causes subjects to strongly feel unwillingness, sense of burden, and the like against sample collection.

Therefore, development of an analysis method that allows measurement of a plurality of types of steroid hormones with a small amount of a body hair sample has been demanded.

### Citation List

### Patent Literature

Patent Literature 1: Japanese Patent No. 4634913 Non Patent Literature
Non Patent Literature 1: Journal of Steroid Biochemistry & Molecular Biology, 162, 92-99, 2016
Non Patent Literature 2: Job Stress Research, 24, 213-218, 2017

### Summary of Invention

An object of the present invention is to provide a method that allows analysis of a plurality of types of steroid hormones with a smaller amount of an animal body hair sample compared with conventional analysis methods. Another object of the present invention is, for example, to provide a method for evaluating the stress of a subject with a slight amount of the hair of the subject.

The present inventors have found that use of a 45 vol% to 55 vol% aqueous acetonitrile solution containing 0.1 M trifluoroacetic acid (TFA) as an extraction solvent allows analysis of a plurality of types of steroid hormones from a smaller amount of an animal body hair sample compared with conventional methods. Further, the present inventors have invented a method for stress evaluation and a method for analyzing a cause of hair loss which use this analysis method.

The present invention provides the followings.
[1] An analysis method for analyzing steroid hormones contained in a body hair sample from an animal, wherein
   body hair collected from an animal is used as the body hair sample, and the analysis method includes:
      an extraction step of extracting a plurality of types of steroid hormones from the body hair sample by using a 45 vol% to 55 vol% aqueous acetonitrile solution containing 0.1 M trifluoroacetic acid; and
   a measurement step of measuring the amounts of the plurality of types of steroid hormones extracted.
[2] The analysis method according to [1], comprising a pulverizing step of pulverizing the body hair sample before the extraction step.
[3] The analysis method according to [1] or [2], wherein, in the measurement step, the amounts of the plurality of types of steroid hormones are measured by using liquid chromatography, gas chromatography, electrophoresis, radioimmunoassay, enzyme immunoassay, mass spectrometry, or a combination of two or more thereof.
[4] The analysis method according to any one of [1] to [3], wherein the amount of the body hair sample is 0.35 mg or more.
[5] The analysis method according to any one of [1] to [3], wherein the amount of the body hair sample is 0.35 mg to 3.5 mg.
[6] The analysis method according to any one of [1] to [3], wherein the amount of the body hair collected from an animal is two or more strands of body hair.
[7] The analysis method according to any one of [1] to [3], wherein the amount of the body hair collected from an animal is 2 to 20 strands of body hair.
[8] The analysis method according to any one of [1] to [7], wherein the plurality of types of steroid hormones are three or more types of steroid hormones.
[9] The analysis method according to any one of [1] to [8], wherein the plurality of types of steroid hormones is selected from the group consisting of a glucocorticoid, a sex hormone, and a combination thereof.
[10] The analysis method according to any one of [1] to [9], wherein the body hair sample is body hair collected from a human.
[11] The analysis method according to [10], wherein the body hair sample is body hair collected from the parietal region, preferably the center of the parietal region, of a human head, and the plurality of types of steroid hormones includes a sex hormone, preferably DHT.
[12] A method for stress evaluation, comprising:
   a step of obtaining 2 to 20 strands of hair of a subject from the root of the hair;
   an extraction step of extracting at least two types of steroid hormones selected from cortisol, cortisone, and DHEA from the hair by using a 45 vol% to 55 vol% aqueous acetonitrile solution containing 0.1 M trifluoroacetic acid;
   a measurement step of measuring the amounts of the at least two types of steroid hormones extracted; and
   a step of reading out measurement results for a plurality of control subjects from a storage unit and evaluating the stress of the subject by comparison with the measurement results read out.
[13] A method for analyzing a cause of hair loss, comprising:
   a step of obtaining 2 to 20 strands of hair of a subject from the root of the hair;
   an extraction step of extracting a first steroid hormone being at least one selected from cortisol, cortisone, and DHEA, and a second steroid hormone being at least one selected from DHT and testosterone, from the hair by using a 45 vol% to 55 vol% aqueous acetonitrile solution containing 0.1 M trifluoroacetic acid;
   a measurement step of measuring the amounts of the first steroid hormone extracted and the second steroid hormone extracted; and
   a step of analyzing the cause of hair loss by comparing the amounts of the first steroid hormone with those of the second steroid hormone.
[14] A method for stress evaluation, comprising:
   a step of obtaining previous physical data for a subject;
   a step of obtaining hair of the subject from the root of the hair;
   a step of cutting the hair of the subject into a plurality of sections specified by distance from the root, and analyzing the amount of at least one steroid hormone selected from cortisol, cortisone, and DHEA contained in each cut section; and
   a step of specifying the time of generation of each section of the hair on the basis of the distance, and outputting the previous physical data and an analysis result for the at least one steroid hormone in relation to each other in a time series.
[15] A method for stress evaluation, comprising:
   a step of obtaining previous attendance or schedule data for a subject;
   a step of obtaining hair of the subject from the root of the hair;
   a step of cutting the hair of the subject into a plurality of sections specified by distance from the root, and analyzing the amount of at least one steroid hormone selected from cortisol, cortisone, and DHEA contained in each cut section; and
   a step of specifying the time of generation of each section of the hair on the basis of the distance, and outputting the previous attendance or schedule data and an analysis result for the at least one steroid hormone in relation to each other in a time series.
[16] A method for measuring training effect, comprising:
   a step of obtaining previous training data for a subject;
   a step of obtaining hair of the subject from the root of the hair;
   a step of cutting the hair of the subject into a plurality of sections specified by distance from the root, and analyzing the amount of at least one steroid hormone selected from DHT, testosterone, and cortisol contained in each cut section; and
   a step of specifying the time of generation of each section of the hair on the basis of the distance, and outputting the previous training data and an analysis result for the at least one steroid hormone in relation to each other in a time series.
[17] A method for stress evaluation, comprising:
   a step of obtaining hair of a plurality of subjects and data on a group to which the subjects belong;
   a step of collecting the hair of a plurality of subjects belonging to the same group, and analyzing the amount of at least one steroid hormone selected from cortisol, cortisone, and DHEA; and
   a step of outputting an analysis result for the at least one steroid hormone.

### Effects of Invention

The analysis method of the present invention allows analysis of a plurality of types of steroid hormones from a smaller amount of body hair compared with conventional analysis methods.

### Brief Description of Drawings

[Figure 1] Figure 1 is a diagram illustrating the configuration of a stress evaluation apparatus of a first embodiment.
[Figure 2] Figure 2 is a flowchart illustrating a method for stress evaluation of the first embodiment.
[Figure 3] Figure 3 is a diagram illustrating the configuration of a hair loss cause analysis apparatus of a second embodiment.
[Figure 4] Figure 4 is a flowchart illustrating a method for analyzing a cause of hair loss of the second embodiment.
[Figure 5] Figure 5 is a diagram illustrating the configuration of a stress evaluation apparatus of a third embodiment.
[Figure 6] Figure 6 is a flowchart illustrating a method for stress evaluation of the third embodiment.
[Figure 7] Figure 7 is a flowchart illustrating a method for stress evaluation of a fourth embodiment.
[Figure 8] Figure 8 is a diagram illustrating the configuration of a training effect measurement apparatus of a fifth embodiment.
[Figure 9] Figure 9 is a flowchart illustrating a method for measuring training effect of the fifth embodiment.
[Figure 10] Figure 10 is a flowchart illustrating a method for stress evaluation of a sixth embodiment.

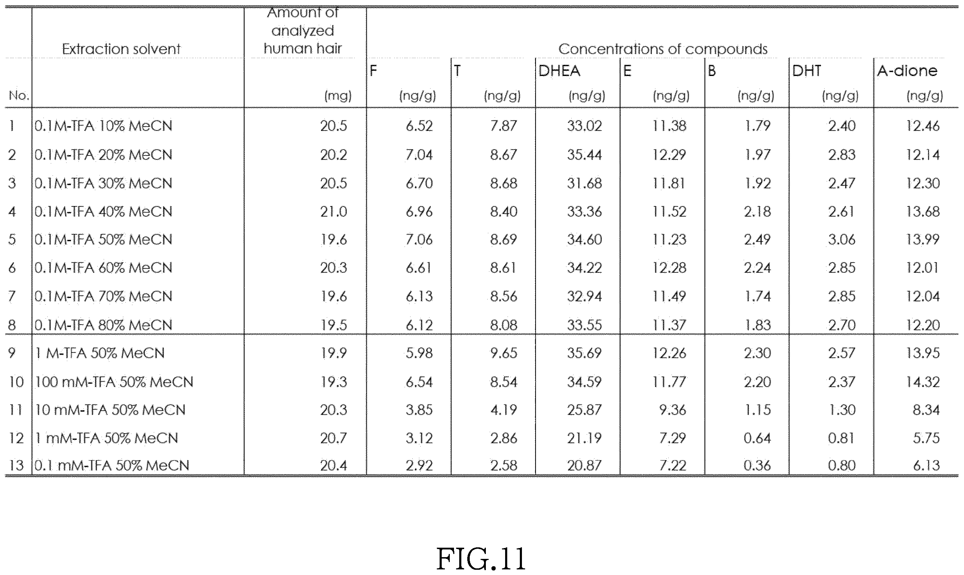

[Figure 11] Figure 11 shows data of concentrations of steroid hormones in hair samples as measured in Example 1; in the figure, F represents cortisol; T represents testosterone; DHEA represents dehydroepiandrosterone; E represents cortisone; B represents corticosterone; DHT represents dihydrotestosterone; and A-dione represents androstenedione. (The same is applied to subsequent figures). As an extraction solvent, a 10 vol% aqueous acetonitrile solution was used for specimen No. 1, a 20 vol% aqueous acetonitrile solution was used for specimen No. 2, a 30 vol% aqueous acetonitrile solution was used for specimen No. 3, a 40 vol% aqueous acetonitrile solution was used for specimen No. 4, a 50 vol% aqueous acetonitrile solution was used for specimen No. 5, a 60 vol% aqueous acetonitrile solution was used for specimen No. 6, a 70 vol% aqueous acetonitrile solution was used for specimen No. 7, and an 80 vol% aqueous acetonitrile solution was used for specimen No. 8 (irrespective of the acetonitrile concentration, the aqueous acetonitrile solutions each contain 0.1 M trifluoroacetic acid). Fifty vol% aqueous acetonitrile solutions containing 1000 mM trifluoroacetic acid, 100 mM trifluoroacetic acid, 10 mM trifluoroacetic acid, 1 mM trifluoroacetic acid, and 0.1 mM trifluoroacetic acid were used as extraction solvents for specimen No. 9, specimen No. 10, specimen No. 11, specimen No. 12, and specimen No. 13, respectively.
[Figure 12] Figure 12 is a graph representing the relationship between acetonitrile concentrations and extraction rates for cortisol, cortisone, and corticosterone on the basis of the data in Figure 11; in the graph, an extraction rate with 50 vol% aqueous acetonitrile solution (containing 0.1 M TFA) is assumed to be 100%.
[Figure 13] Figure 13 is a graph representing the relationship between acetonitrile concentrations and extraction rates for testosterone, dihydrotestosterone, androstenedione, and dehydroepiandrosterone on the basis of the data in Figure 11; in the graph, an extraction rate with 50 vol% aqueous acetonitrile solution (containing 0.1 M TFA) is assumed to be 100%.
[Figure 14] Figure 14 is a graph representing the relationship between TFA concentrations and extraction rates for cortisol, cortisone, and corticosterone on the basis of the data in Figure 11; in the graph, an extraction rate with 50 vol% aqueous acetonitrile solution having a TFA concentration of 100 mM is assumed to be 100%.
[Figure 15] Figure 15 is a graph representing the relationship between TFA concentrations and extraction rates for testosterone, dihydrotestosterone, androstenedione, and dehydroepiandrosterone on the basis of the data in Figure 11; in the graph, an extraction rate with 50 vol% aqueous acetonitrile solution having a TFA concentration of 100 mM is assumed to be 100%.

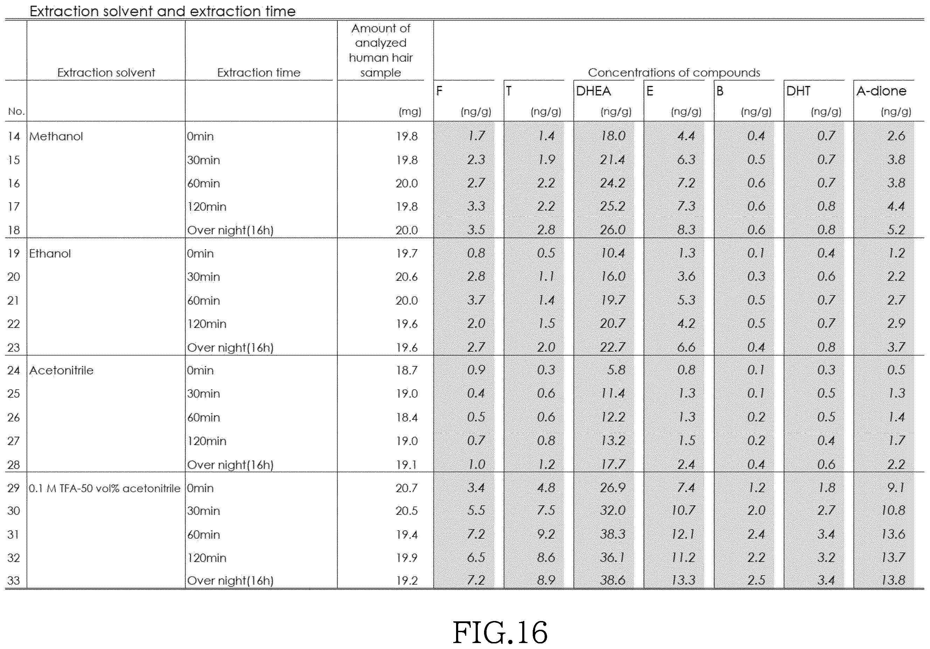

[Figure 16] Figure 16 shows data of concentrations of steroid hormones in hair samples as measured in Example 2. As the extraction solvent, methanol was used for specimen Nos. 14 to 18, ethanol was used for specimen Nos. 19 to 23, acetonitrile was used for specimen Nos. 24 to 28, and a 50 vol% aqueous acetonitrile solution containing 0.1 M trifluoroacetic acid was used for specimen Nos. 29 to 33. The extraction time was 0 minute for specimen Nos. 14, 19, 24, and 29, 30 minutes for specimen Nos. 15, 20, 25, and 30, 60 minutes for specimen Nos. 16, 21, 26, and 31, 120 minutes for specimen Nos. 17, 22, 27, and 32, and 16 hours (overnight) for specimen Nos. 18, 23, 28, and 33.
[Figure 17] Figure 17 is a graph representing the data of cortisol in Figure 16.
[Figure 18] Figure 18 is a graph representing the data of testosterone in Figure 16.
[Figure 19] Figure 19 is a graph representing the data of dehydroepiandrosterone in Figure 16.
[Figure 20] Figure 20 shows data of concentrations of steroid hormones in hair samples as measured in Example 3.
[Figure 21] Figure 21 is a diagram illustrating nine sites from which hair was collected in Example 4.
[Figure 22] Figure 22 shows data of dihydrotestosterone concentrations in hair samples as measured in Example 4.
[Figure 23] Figure 23 shows data of cortisol concentrations in hair samples as measured in Example 4.
[Figure 24] Figure 24 is graphs representing Z-scores of concentrations of steroid hormones at different sites of head as measured in Example 4.
[Figure 25] Figure 25 shows data of concentrations of steroid hormones in hair samples as measured in Example 5.

### Description of Embodiments

Hereinafter, the present invention will be described with reference to preferred embodiments. First, an analysis method is described, and then a method for stress evaluation and others using the analysis method are described. Thereafter, Examples of the analysis method are described.

Herein, "steroid hormones" are hormones having a steroid skeleton, and include sex hormones (male hormones and female hormones), glucocorticoids, mineral corticoids (mineralocorticoids), and vitamin D₃ analogs.

The analysis method of the present invention allows analysis of various steroid hormones. Through combined analysis or comprehensive analysis of multiple steroid hormones, stress, alopecia, aging, metabolic disorder, and other physical or mental conditions in subject animals can be evaluated or monitored. The analysis method of the present invention can be utilized for doping test for humans. It is preferable that the number of steroid hormones to be analyzed be two or more, three or more, four or more, five or more, six or more, seven or more, eight or more, nine or more, or 10 or more. For example, the number of steroid hormones to be analyzed may be two to seven, or six or seven. Examples of the steroid hormones to be analyzed include, but are not limited to, glucocorticoids such as cortisone, cortisol, and corticosterone, male hormones such as testosterone, dihydrotestosterone, dehydroepiandrosterone, androstenedione, and androstenediol, and female hormones such as progesterone. These steroid hormones can be selected in appropriate combinations to suit the purpose. For example, the steroid hormones to be analyzed may be cortisone, cortisol, corticosterone, dehydroepiandrosterone and the like for the purpose of examining stress conditions, testosterone, dihydrotestosterone and the like for the purpose of examining on alopecia, and various sex hormones, active vitamin D₃ and the like for the purpose relating to antiaging. Alternatively, health conditions in subject animals may be comprehensively evaluated or monitored by analyzing steroid hormones in total.

Herein, the "animal" in "a body hair sample from an animal" is not limited, and for example, mammals, birds, and insects are included in the scope. The body hair of a mammal such as a human, a monkey, a dog, a cat, a bovine, a horse, a pig, a rat, and a mouse is preferably used as "a body hair sample from an animal".

The analysis method of the present invention will be specifically described below; however, the present invention is not limited to the following specific Embodiments or Examples.

### <Body Hair Sample>

The amount of a body hair sample to be used for the analysis method of the present invention is at least one or more strands of hair, and preferably two or more strands of hair, though the amount may depend on the detection sensitivity of measurement apparatus. For example, the amount of a body hair sample may be two, three, four, five, six, seven, eight, nine, 10, 11, 12, 13, 14, 15, or 16 strands of hair. The amount of a body hair sample may be more than 20 strands of hair. Two to twenty, 3 to 17, 4 to 15, or 5 to 12 strands of hair may be used. The amount of a body hair sample is at least 0.1 mg or more, and preferably 0.35 mg or more. For example, the amount of a body hair sample may be 0.5 mg, 0.6 mg, 0.7 mg, 0.8 mg, or more. The amount of a body hair sample may be less than 0.8 mg, 1 mg, 1.5 mg, 2 mg, 2.5 mg, 3 mg, or 3.5 mg. For example, the amount of a body hair sample may be 0.35 mg to 1 mg, 0.35 mg to 1.5 mg, 0.35 mg to 2 mg, 0.35 mg to 2.5 mg, 0.35 mg to 3 mg, 0.35 mg to 3.5 mg, 0.5 mg to 1 mg, 0.5 mg to 1.5 mg, 0.5 mg to 2 mg, 0.5 mg to 2.5 mg, 0.5 mg to 3 mg, 0.5 mg to 3.5 mg, 0.8 mg to 1 mg, 0.8 mg to 1.5 mg, 0.8 mg to 2 mg, 0.8 mg to 2.5 mg, 0.8 mg to 3 mg, or 0.8 mg to 3.5 mg. For a body hair sample from a human, hair, pubic hair, or other hair can be used. If human hair is used as a body hair sample, the hair can be collected, for example, from the frontal region, the parietal region, the occipital region, or the temporal region of head. For example, the hair may be collected from the occipital region (e.g., the center of the occipital region), or the parietal region (e.g., the center of the parietal region). For the purpose of analyzing hormones relating to AGA (e.g., sex hormones such as DHT), it is preferred to collect the hair from the parietal region, and it is more preferred to collect the hair from the center of the parietal region. Analysis of hormones relating to AGA can be utilized for evaluating or monitoring AGA.

If a section of body hair in a given length from the root of the hair is analyzed as a sample, the amounts of steroid hormones accumulated in the period corresponding to the length can be clarified. For example, human hair typically grows by about 1 mm in 3 days, and thus if a section of human hair ranging from the hair root to a position 3 cm distant from the hair root is analyzed as a sample, the amounts of steroid hormones accumulated in approximately 3 months can be revealed. Alternatively, if a plurality of sections of a strand of body hair is cut out as samples, changes in steroid hormone levels in the past can be analyzed.

### <Washing>

In order to remove substances (e.g., sweat, fat, dusts) adhering to the outside of the collected body hair, it is preferable to wash the body hair before extracting steroid hormones therefrom. Water or an organic solvent may be appropriately used as washing liquids. For example, 2-propanol may be preferably used, and methanol, chloroform, dichloromethane, and the like may be also used.

Before being subjected to the extraction step, the washed body hair sample may be ground as appropriate with a common method such as a ball mill, or may be ground as appropriate together with an extraction solvent. Alternatively, the washed body hair sample may be directly subjected to the extraction step without being ground.

### <Extraction of Steroid Hormones from Body Hair Sample>

The ground (or not ground) body hair sample is incubated in an aqueous acetonitrile solution containing 0.1 M TFA for 30 minutes to 16 hours to extract steroid hormones. It is preferable that the concentration of the aqueous acetonitrile solution be 45 vol% to 55 vol%. The duration of the extraction is preferably about 30 minutes to 2 hours, and more preferably 1 hour from the viewpoint of reducing the analysis time.

The extract obtained may be directly subjected to measurement; or, steroid hormones in the extract may be purified by appropriately selecting common preparation methods such as separation and purification methods with column chromatography or the like before being subjected to measurement.

Steroid hormones may be converted into ester derivatives such as picolinate derivatives before being subjected to measurement. In general, this reaction can be performed for steroid hormones dissolved or suspended in an inert organic solvent, for example, an ether such as dioxane, tetrahydrofuran, and dimethoxyethane, an aromatic hydrocarbon such as benzene, toluene, and xylene, an amide such as dimethylformamide and dimethylacetamide, acetonitrile, or dimethyl sulfoxide, in the presence of an appropriate base such as sodium hydride, potassium carbonate, triethylamine, and pyridine. The reaction temperature can be set within the range of -5°C to 80°C in typical cases, and preferably temperatures in the range of 10°C to 30°C are suitable. The duration of the reaction time can be set within the range of 5 to 240 minutes in typical cases, and preferably, the duration in a range of 30 to 90 minutes is suitable.

The ratio of the usage of the derivatization reagent to the amount of the sample in the ester derivatization reaction is not limited, and, in typical cases, at least 0.2 mg, and preferably in the range of 1 to 5 mg of a compound to serve as the derivatization reagent can be used per mL of the sample. The amount of the base to be used in the ester derivatization reaction is not limited, and can be at least 0.1 mmol, and preferably in the range of 0.1 to 0.5 mmol per mL of the sample in typical cases.

If steroid hormones are subjected to ester derivatization as described above, they are then subjected to post-treatment with a conventional method, followed by measurement.

### <Measurement of Steroid Hormones>

For measurement of the amounts of steroid hormones, liquid chromatography, gas chromatography, electrophoresis, radioimmunoassay, enzyme immunoassay, mass spectrometry, and the like can be used. Two or more of them may be used in combination. In order to analyze a plurality of types of steroid hormones with a small amount of a body hair sample, use of a measurement method of high sensitivity is preferred, and measurement may be performed, for example, with a combination of liquid chromatography and tandem mass spectrometry (LC-MS/MS). Two-dimensional liquid chromatography (2D-LC) may be used as the liquid chromatography.

Common apparatuses can be used for liquid chromatography, gas chromatography, electrophoresis, radioimmunoassay, enzyme immunoassay, mass spectrometry, and the like. For example, an Agilent 1290 Infinity LC system manufactured by Agilent Technologies may be used for liquid chromatography, and an MS such as an API5000 manufactured by AB Sciex Pte. Ltd. may be used for mass spectrometry.

Next, a method for stress evaluation and a method for analyzing a cause of hair loss using the thus-described analysis method will be described.

### (First Embodiment)

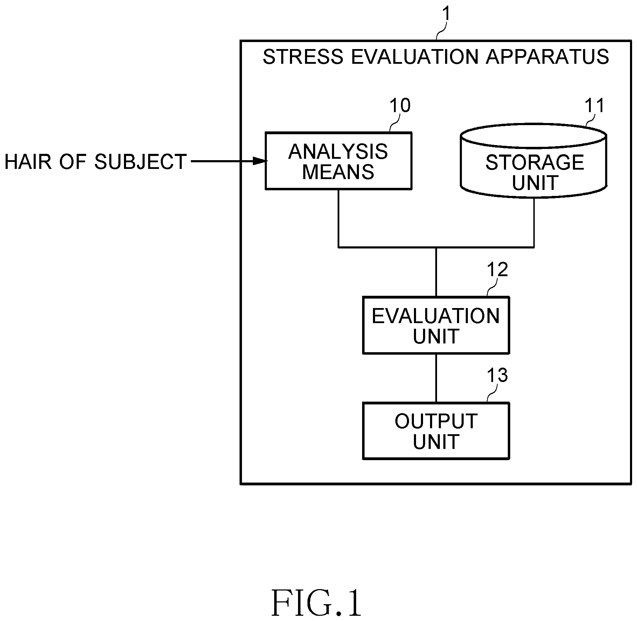

Figure 1 is a diagram illustrating the configuration of a stress evaluation apparatus 1 of the first embodiment. The stress evaluation apparatus 1 includes: an analysis means 10 that performs the above-described analysis method; a storage unit 11 that stores measurement results on stress for control subjects; an evaluation unit 12 that evaluates the stress of a subject by comparing an analysis result from the analysis means 10 and the measurement results for control subjects; and an output unit 13 that outputs an evaluation result.

The analysis means 10 is a collective entity of apparatuses to perform washing and pulverizing of hair, extraction and purification of steroid hormones, and measurement of the amounts of steroid hormones. Details of apparatuses to perform the individual steps of analysis are as described above.

The measurement results for control subjects stored in the storage unit 11 are measurement results on stress for numerous subjects. The average stress level can be understood by determining the average value of numerous measurement results, and comparison with the average stress level allows evaluation on whether the stress level in a subject is high or low.

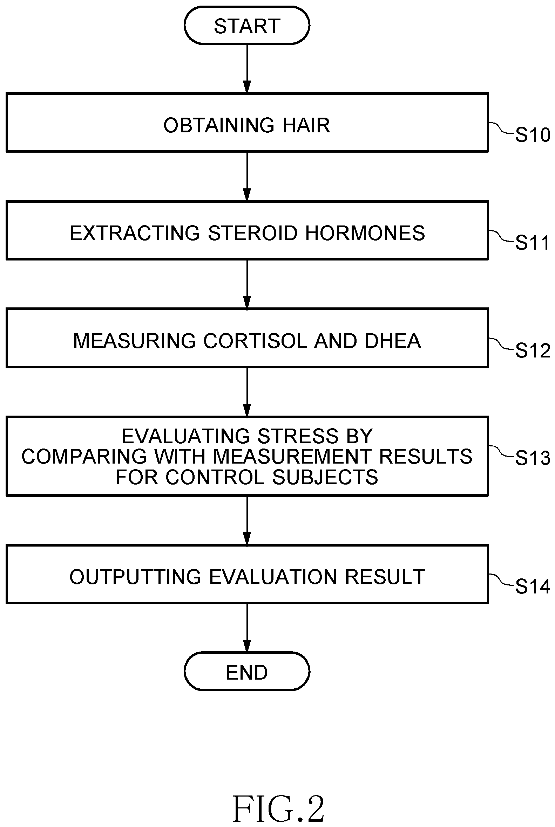

Figure 2 is a flowchart illustrating a method for stress evaluation with the stress evaluation apparatus 1 of the first embodiment. First, a subject him- or herself or a user of the stress evaluation apparatus 1 obtains 2 to 20 strands of hair from the subject (S10). For example, the hair is collected by cutting it at a position as close to the scalp as possible with scissors.

Subsequently, the hair of the subject is incubated in a 45 vol% to 55 vol% aqueous acetonitrile solution containing 0.1 M TFA to extract steroid hormones (S11). Next, the amounts of cortisol and DHEA are measured by using the analysis means 10 (S12), and the data on cortisol and DHEA measured with the analysis means 10 is inputted to the evaluation unit 12. Although an example in which cortisol and DHEA are measured as steroid hormones relating to stress is shown here, the steroid hormones relating to stress are not limited to cortisol and DHEA. For example, cortisone may be used in place of cortisol, and cortisone may be used in addition to cortisol and DHEA.

The evaluation unit 12 reads out measurement results for control subjects from the storage unit 11, and evaluates the stress of the subject by comparing the average values of the measurement results read out with the amounts of cortisol and DHEA in the subject (S13). If the amount of cortisol is larger than the average value of the measurement results for control subjects, the stress of the subject can be evaluated to be higher than the average. Because one of the effects of DHEA is anti-glucocorticoid effect, the stress of the subject is evaluated, for example, by using the ratio between cortisol and DHEA. Thus, the evaluation unit 12 evaluates the stress of the subject by comparing the amounts of multiple types of steroid hormones relating to stress with the measurement results for control subjects. The output unit 13 outputs data on the stress of the subject that results from the evaluation by the evaluation unit 12.

The stress measurement apparatus and stress measurement method of the first embodiment has been described above. The method for stress measurement of the present embodiment, which employs the analysis method as described above, allows proper evaluation of the stress of a subject by measuring the amounts of multiple types of stress hormones with as few as 2 to 20 strands of hair.

It is natural to be unwilling to provide much hair for the purpose of stress test. The above-described analysis method has enabled measurement of a plurality of types of steroid hormones relating to stress with a few strands of hair; this is why the method for stress evaluation of the present embodiment has become possible.

### (Second Embodiment)

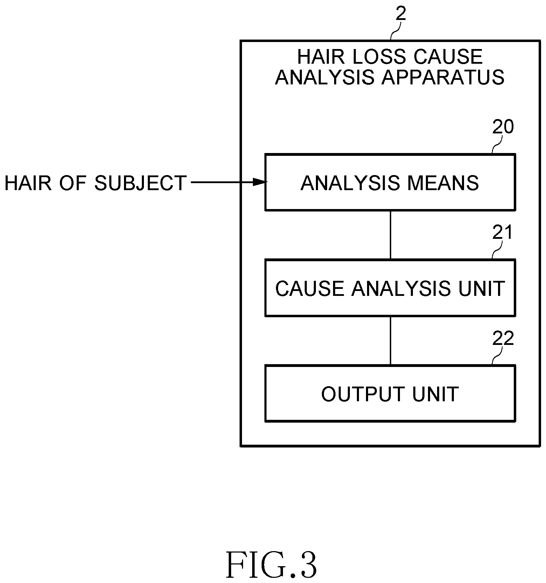

Figure 3 is a diagram illustrating the configuration of a hair loss cause analysis apparatus 2 according to the second embodiment. The hair loss cause analysis apparatus 2 includes: an analysis means 20 that performs the above-described analysis method; a cause analysis unit 21 that analyzes the cause of hair loss on the basis of the analysis result; and an output unit 22 that outputs the analysis result. By inference, causes of hair loss are broadly divided into male hormone-induced hair loss and stress-induced hair loss. The hair loss cause analysis apparatus 2 of the present embodiment analyzes the cause of hair loss on the basis of steroid hormones contained in the hair of a subject.

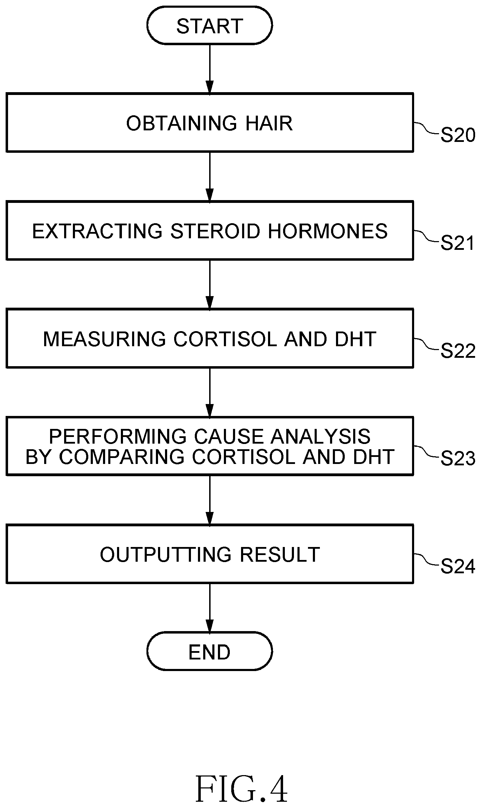

Figure 4 is a flowchart illustrating an analysis method for the cause of hair loss with the hair loss cause analysis apparatus 2 of the second embodiment. First, a subject him- or herself or a user of the hair loss cause analysis apparatus obtains 2 to 20 strands of hair from the subject (S20). For example, the hair is collected by cutting it at a position as close to the scalp as possible with scissors.

Subsequently, the hair of the subject is incubated in a 45 vol% to 55 vol% aqueous acetonitrile solution containing 0.1 M TFA to extract steroid hormones (S21). Next, the amounts of cortisol and DHT are measured by using the analysis means 20 (S22). Cortisol is a steroid hormone relating to stress, and DHT is a steroid hormone relating to male hormone. The analysis means 20 inputs data on cortisol and DHT measured to the cause analysis unit 21.

The cause analysis unit 21 analyzes which of stress and male hormone is the cause of hair loss on the basis of the amounts of cortisol and DHT in the hair of the subject (S23). If the amount of cortisol is relatively larger than that of DHT, the analysis determines that stress is the cause of hair loss. If the amount of DHT is relatively larger than that of cortisol, the analysis determines that male hormone is the cause of hair loss.

The cause analysis unit 21 may use data for control subjects, as in the first embodiment. For example, deviation scores of cortisol and DHT contained in the hair of the subject may be calculated on the basis of the distribution of the measurement results for control subjects for analysis to determine the compound with the higher deviation score to be a contributor to the cause. The hair loss cause analysis apparatus 2 outputs the cause of hair loss determined by the cause analysis unit 21 from the output unit 22 to present it to the subject or a physician (S24).

The hair loss cause analysis apparatus 2 and the method for analyzing a cause of hair loss of the second embodiment has been described above. The method for analyzing a cause of hair loss of the present embodiment allows evaluation on which of stress and male hormone is the cause of hair loss in a subject through measurement of the amounts of cortisol and DHT with as few as 2 to 20 strands of hair.

While the cause of hair loss in a subject is analyzed on the basis of the amounts of cortisol and DHT contained in the hair of the subject in the present embodiment, cortisone or DHEA may be used as a steroid hormone relating to stress, and cortisone or DHEA may be used in addition to cortisol. Testosterone or both DHT and testosterone may be used as a steroid hormone(s) relating to male hormone. Although a subject having a symptom of hair loss has been shown as an example in the description of the present embodiment, analysis of steroid hormones may be performed for a subject having no symptom of hair loss to prevent hair loss.

### (Third Embodiment)

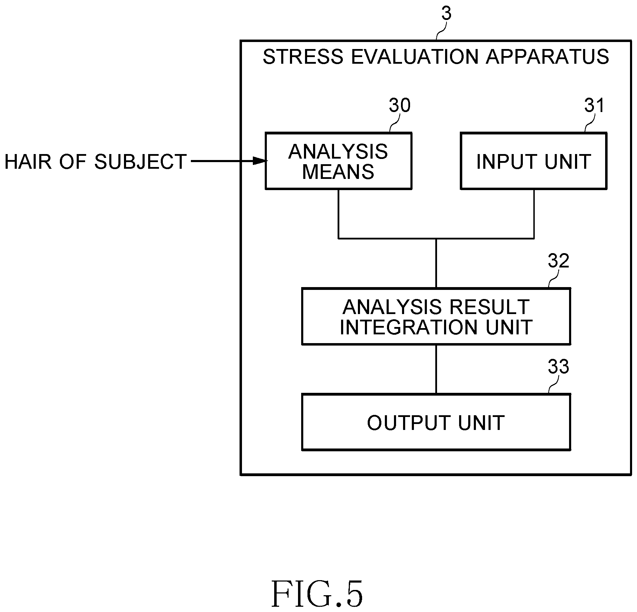

Figure 5 is a diagram illustrating the configuration of a stress evaluation apparatus 3 of the third embodiment. The stress evaluation apparatus 3 includes: an analysis means 30 that performs the above-described analysis method; an input unit 31 that receives inputs of physical data for a subject; an analysis result integration unit 32 that integrates analysis results from the analysis means 30 and the physical data for a subject that has been inputted from the input unit 31 and generates an analysis result; and an output unit 33 that outputs the analysis result.

The analysis means 30 is a collective entity of apparatuses to perform the above-described washing and pulverizing of hair, extraction and purification of steroid hormones, and measurement of the amounts of steroid hormones.

The physical data the inputs of which are to be received by the input unit 31 includes body weight, body fat, blood pressure, sleeping hours, and walking steps. The physical data is not data at a certain time point, but time-series data, which is at least data for a period of the last 1 month, data for a period from 2 months before to 1 month before, or data for a period from 3 months before to 2 months before. If a plurality of sets of data is present for a period of interest, representative data may be selected on the basis of dates of measurement; alternatively, the average values may be calculated.

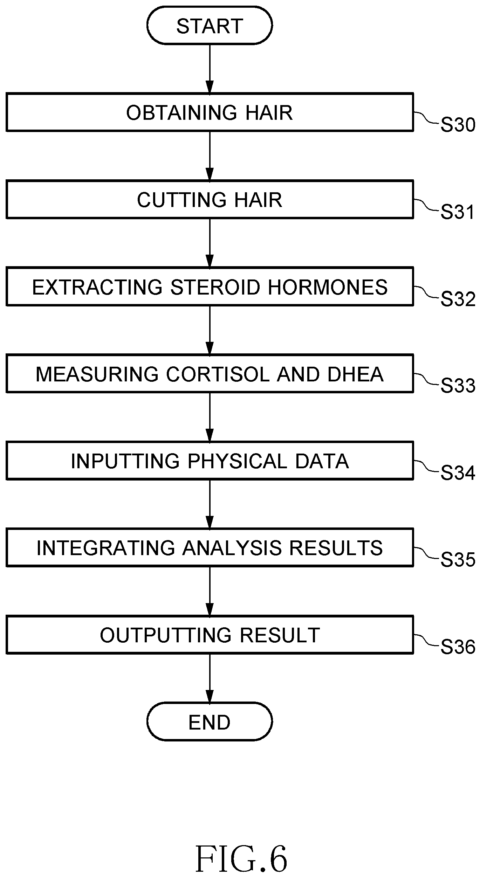

Figure 6 is a flowchart illustrating a method for stress evaluation with the stress evaluation apparatus 3 of the third embodiment. First, a subject him- or herself or a user of the stress evaluation apparatus 3 obtains 2 to 20 strands of hair from the subject (S30). For example, the hair is cut out as close to the scalp as possible with scissors to collect it.

Next, the hair of the subject is cut at 1-cm intervals from the hair root (S31). Since the hair grows by about 1 cm in a month, it can be said that a section of hair ranging from the root of the hair to a position 1 cm distant from the root was generated in a period of the last about 1 month, a section of the hair ranging from a position 1 cm distant from the root to a position 2 cm distant from the root was generated in a period from approximately 2 months before to approximately 1 month before, and a section of the hair ranging from a position 2 cm distant from the root to a position 3 cm distant from the root was generated in a period from approximately 3 months before to approximately 2 months before. In the present embodiment, the hair of the subject from the root to a position 3 cm distant from the root is cut at 1-cm intervals to divide into three sections.

Subsequently, analysis of the hair of the subject is performed by using the analysis means 30, wherein the cut sections of the subject's hair are subjected to the analysis in the present embodiment. Specifically, the section ranging from the root to a position 1 cm distant from the root, the section ranging from a position 1 cm distant from the root to a position 2 cm distant from the root, and the section ranging from a position 2 cm distant from the root to a position 3 cm distant from the root are individually subjected to the analysis. The method of analysis is the same as that in the first embodiment: the hair of the subject is incubated in a 45 vol% to 55 vol% aqueous acetonitrile solution containing 0.1 M TFA to extract steroid hormones by using the analysis means 30 (S32).

Next, the amounts of cortisol and DHEA are measured by using the analysis means 30 (S33), and the data on cortisol and DHEA measured with the analysis means 30 is inputted to the analysis result integration unit 32.

The stress evaluation apparatus 3 receives inputs of physical data including the body weight and blood pressure of the subject from the input unit 31 (S34). The input unit 31 passes the inputted physical data to the analysis result integration unit 32.

Next, the analysis result integration unit 32 integrates analysis results for the sections of the hair of the subject and the physical data for the subject that have been inputted from the input unit 31 (S35). Specifically, the data on cortisol and DHEA extracted from the section of the hair ranging from the root to a position 1 cm distant from the root and the physical data for a period of the last 1 month are related to each other. Similarly, the measurement result for the section ranging from a position 1 cm distant from the root to a position 2 cm distant from the root and the physical data for a period from 2 months before to 1 month before are related to each other, and the measurement result for the section ranging from a position 2 cm distant from the root to a position 3 cm distant from the root and the physical data for a period from 3 months before to 2 months before are related to each other. The output unit 33 outputs data on the stress of the subject that has been obtained through the integration with the physical data by the analysis result integration unit 32 (S36).

The method for stress evaluation of the third embodiment has been described above. The method for stress evaluation of the present embodiment allows the relationship between physical data and stress levels to be understood by outputting analysis results on cortisol or cortisone and DHEA, which are steroid hormones relating to stress, in relation to physical data. For example, once increased blood pressure is revealed to be a characteristic of physical data in a period with increased stress levels, the blood pressure can be controlled to be low by performing a means to eliminate stress (e.g., taking enough rest, taking light exercise).

Although a simple example in which the analysis result integration unit 32 relates analysis results on cortisol to DHEA and physical data with respect to their time series and outputs the result has been described for the present embodiment, factors of physical data relating to cortisol or DHEA may be determined through regression analysis or the like to present the factors to the subject.

Although an example in which cortisol and DHEA are measured as steroid hormones relating to stress has been shown for the present embodiment, the steroid hormones relating to stress are not limited to cortisol and DHEA. For example, cortisone may be used in place of cortisol, and cortisone may be used in addition to cortisol and DHEA. Alternatively, one steroid hormone may be used for the method for stress evaluation of the present embodiment. In this case, the steroid hormone in the hair of a subject may be analyzed with an analysis method differing from the analysis method described for the present embodiment.

### (Fourth Embodiment)

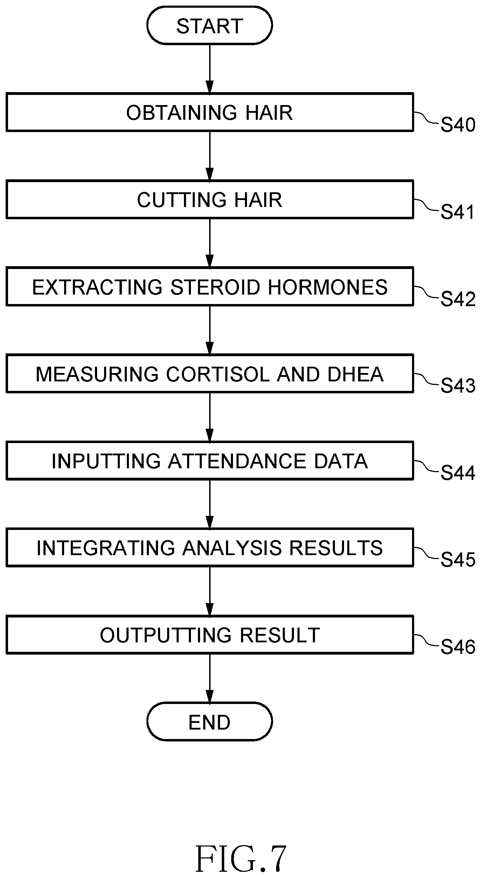

Figure 7 is a flowchart illustrating a method for stress evaluation of the fourth embodiment. In the method for stress evaluation of the fourth embodiment, measurement results obtained through analysis of the hair of a subject are related to attendance data for the subject, and the result is outputted. The basic configuration of an apparatus for use in the method for stress evaluation of the fourth embodiment is the same as that of the stress evaluation apparatus of the third embodiment, except that attendance data for a subject is used in place of physical data for a subject, which is used in the third embodiment. In the method for stress evaluation of the fourth embodiment, processes from obtaining the hair of a subject (S40) to measuring cortisol and DHEA (S43) are the same as those in the method for stress evaluation of the third embodiment. In the method for stress evaluation of the fourth embodiment, attendance data for a subject is inputted in step S44, and attendance data and analysis results on stress are integrated month by month in step S45.

For the attendance data, for example, overtime hours can be used. This allows the subject to understand whether overtime hours and stress are related to each other, and if so, how strong the correlation is. In addition to overtime hours, for example, the number of days of paid vacation time used or the number of days of tardiness or early leaving can be used as attendance data. Alternatively, calendar data for a subject can be used in place of attendance data. Calendar data is data on meetings, business trips, travels, and so on. A subject can understand the relationship between calendar data and stress.

### (Fifth Embodiment)

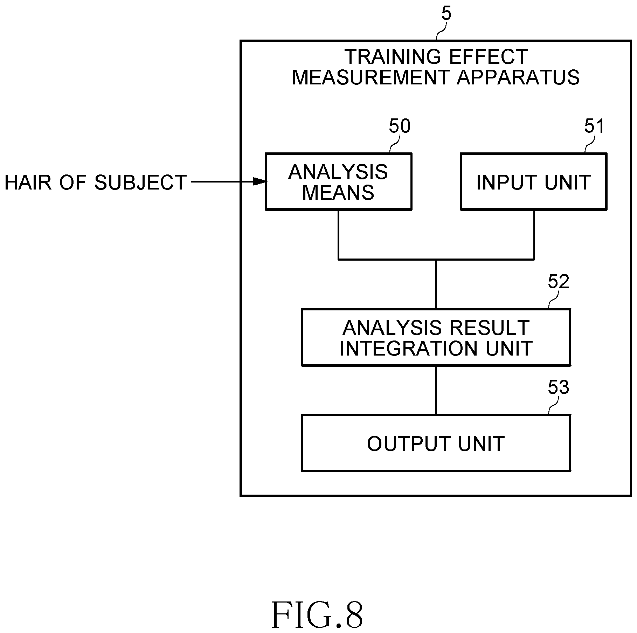

Figure 8 is a diagram illustrating the configuration of a training effect measurement apparatus 5 of the fifth embodiment. The training effect measurement apparatus 5 of the fifth embodiment includes: an analysis means 50 that performs the above-described analysis method; an input unit 51 that receives inputs of training data for a subject; an analysis result integration unit 52 that integrates analysis results from the analysis means 50 and the training data for a subject that has been inputted from the input unit 51 and generates an analysis result; and an output unit 53 that outputs the analysis result.

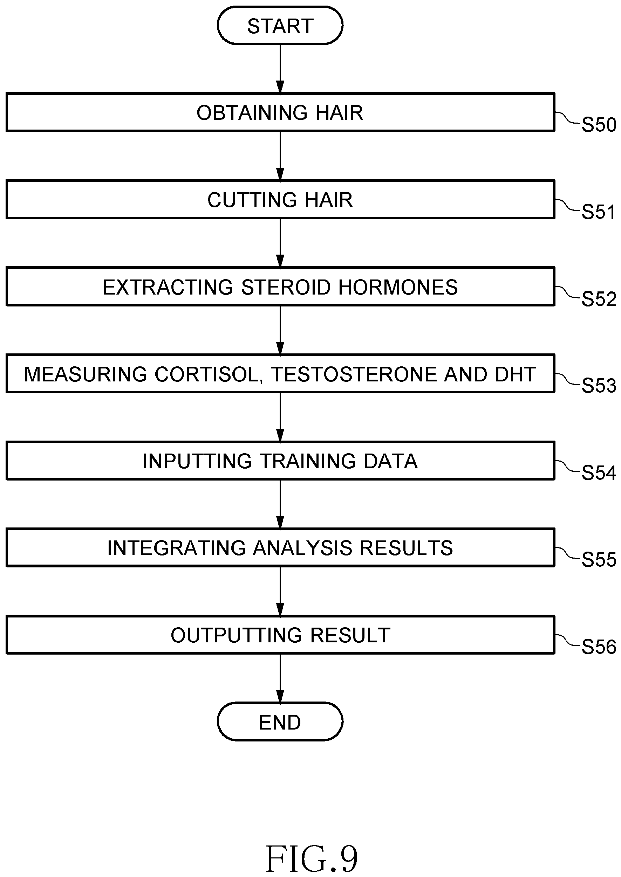

Figure 9 is a flowchart illustrating a training effect measurement method of the fifth embodiment. First, a subject him- or herself or a user of the training effect measurement apparatus 5 obtains 2 to 20 strands of hair from the subject (S50). For example, the hair is cut out as close to the scalp as possible with scissors to collect it. Next, the hair of the subject from the root to a position 3 cm distant from the root is cut at 1-cm intervals to divide into three sections (S51) .

Subsequently, analysis of the hair of the subject is performed, wherein the analysis is performed in terms of distance from the root of the hair in the present embodiment. Specifically, the section of the subject's hair ranging from the root to a position 1 cm distant from the root, the section of the subject's hair ranging from a position 1 cm distant from the root to a position 2 cm distant from the root, and the section of the subject's hair ranging from a position 2 cm distant from the root to a position 3 cm distant from the root are individually subjected to the analysis. The method of analysis is the same as that in the first embodiment: the hair of the subject is incubated in a 45 vol% to 55 vol% aqueous acetonitrile solution containing 0.1 M TFA to extract steroid hormones by using the analysis means 50 (S52). Next, the amounts of cortisol, DHT, and testosterone are measured by using the analysis means 50 (S53), and data on cortisol, DHT, and testosterone measured with the analysis means 50 is inputted to the analysis result integration unit 52.

In the method for measuring training effect, inputs of physical data including the body weight, blood pressure, body fat percentage, and muscle mass of the subject are received from the input unit 51 (S54). The input unit 51 passes the inputted physical data to the analysis result integration unit 52.

The training data the inputs of which are received by the input unit includes the times of training and menus of training. The training data is not data at a certain time point, but time-series data, which is at least data for a period of the last 1 month, data for a period from 2 months before to 1 month before, or data for a period from 3 months before to 2 months before.

Next, the analysis result integration unit 52 integrates analysis results for the sections of the hair of the subject and the training data for the subject that has been inputted from the input unit (S55). Specifically, the data on cortisol, DHT, and testosterone extracted from the section of the hair ranging from the root to a position 1 cm distant from the root and the physical data for a period of the last 1 month are related to each other. Similarly, the measurement result for the section ranging from a position 1 cm distant from the root to a position 2 cm distant from the root and the physical data for a period from 2 months before to 1 month before are related to each other, and the measurement result for the section ranging from a position 2 cm distant from the root to a position 3 cm distant from the root and the physical data for a period from 3 months before to 2 months before are related to each other. The output unit 53 outputs data on the steroid hormones in the subject that has been obtained through the integration with the physical data by the analysis result integration unit 52 (S56).

The training effect measurement method of the fifth embodiment has been described above. By outputting analysis results on DHT and testosterone, which are steroid hormones relating to male hormone, and cortisol, which relates to stress, in relation to training data, the method for measuring training effect of the present embodiment allows the relationship between menus and times of training and those hormones to be understood. Male hormones are known to relate to muscle enhancement and body fat reduction, and cortisol is known to relate to body fat gain and obesity; hence the effect of training can be confirmed on the basis of the amounts of these hormones. In addition, determination can be made on which training menu was effective from the relationship with menus and times of training.

Although an example in which testosterone, DHT, and cortisol are measured as steroid hormones has been shown for the present embodiment, one steroid hormone may be used for the method for measuring training effect of the present embodiment. In this case, the steroid hormone in the hair of a subject may be analyzed with an analysis method differing from the analysis method described for the present embodiment.

### (Sixth Embodiment)

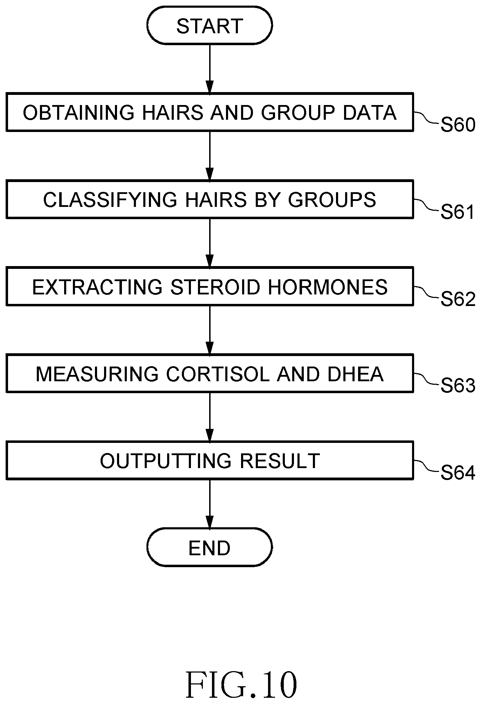

Figure 10 shows a flowchart illustrating a method for stress evaluation of the sixth embodiment. The method for stress evaluation of the sixth embodiment is a method of evaluating stress on group basis by analyzing the hair of a plurality of subjects belonging to the same group in a collective manner.

First, a subject him- or herself or a user of a stress evaluation apparatus obtains hair from the subject (S60). For example, the hair is cut out as close to the scalp as possible with scissors to collect it. In the present embodiment, data for groups to which the subjects belong is obtained together with the hair of the subjects.

Subsequently, the thus-obtained hair of each of the plurality of subjects is classified by the group to which the subject belongs (S61). The groups are based on works or regions. For example, the hair of subjects belonging to the same workplace are combined together.

Subsequently, the hair of the plurality of subjects are incubated in a 45 vol% to 55 vol% aqueous acetonitrile solution containing 0.1 M TFA to extract steroid hormones (S62). Next, the amounts of cortisol and DHEA are measured by using an analysis means (S63). The analysis means outputs data on cortisol and DHEA measured (S64).

Through analysis for hair collected from a plurality of subjects belonging to the same group, the method for stress evaluation of the sixth embodiment can achieve significantly reduced time and effort of analysis, and allows determination of the stress tendency of subjects belonging to the group.

### Examples

Next, Examples of the analysis method of the present invention will be described. Details of standards and internal standards, reagents and columns, apparatuses, and conditions for use of apparatuses are shown in Tables 1 to 5 and 7 to 9.

### (Example 1) Examination on Acetonitrile Concentration and Trifluoroacetic Acid Concentration of Extraction Solvent

To examine the influence of acetonitrile concentration and trifluoroacetic acid concentration on extraction rates for steroid hormones, aqueous acetonitrile solutions with various concentrations and 50 vol% aqueous acetonitrile solutions containing various concentrations of trifluoroacetic acid were used to extract seven types of steroid hormones from a human hair sample.

Seven types of steroid hormones, cortisol, cortisone, corticosterone, testosterone, dehydroepiandrosterone, dihydrotestosterone, and androstenedione, are measured.

### (1) Extraction Solvents Used

As the aqueous acetonitrile solutions with different concentrations, eight kinds of aqueous acetonitrile solutions with the following acetonitrile concentrations were used: 10 vol%, 20 vol%, 30 vol%, 40 vol%, 50 vol%, 60 vol%, 70 vol%, and 80 vol% (each solution contains 0.1 M trifluoroacetic acid). As the 50 vol% aqueous acetonitrile solutions with different concentrations of trifluoroacetic acid, five kinds of 50 vol% aqueous acetonitrile solutions with the following trifluoroacetic acid concentrations were used: 0.1 mM, 1 mM, 10 mM, 100 mM, and 1000 mM.

### (2) Collection and Washing of Hair

Hair samples were collected from three male volunteers and approximately 400 hair strands of them were cut as close to the scalp as possible with scissors. The collected hair strands were pooled together and mixed; approximately 600 mg of the hair was put in a polypropylene tube, and after the addition of 10 mL of 2-propanol, stirred by using a vortex mixer for approximately 3 minutes, then the 2-propanol added was completely discarded. These operations were repeated twice to wash the hair.

### (3) Pulverizing of Hair

The washed hair was put in a polypropylene tube (manufactured by Bio Medical Science, Strong Tube) and crushed (1500 rpm, 4 minutes) into a powder by using a ball mill (manufactured by Bio Medical Science, ShakeMaster Neo/BMS-M10N21), and the pulverized hair was used as a hair sample. This hair sample was used also in Example 2.

### (4) Extraction of Steroid Hormones

Approximately 20-mg aliquots of the hair sample were put in glass spitz tubes, internal standards of the steroid hormones to be measured were added (see Table 1), 0.5 mL of extraction solvent was added, and then incubated at 40°C for 1 hour. After adding 4 mL of methyl tert-butyl ether to the extract, the mixture was shaken. The organic layer was then separated from each of the tubes and evaporated to dryness with a centrifugal evaporator.

### [Table 1]

**Table 1 Standards and internal standards used in Example 1**

| Compounds | Abbreviation | Category | Manufacturer | Loading of internal standard (P⁹) |
|---|---|---|---|---|
| Testosterone | T | standard | Sigma-Aldrich | |
| Testosterone-¹³C₃ | T-¹³C₃ | internal standard | ISO SCIENCE | 400 |
| Dihydrotestosterone | DHT | standard | Sigma-Aldrich | |
| Dihydrotestosterone-¹³C₃ | DHT-¹³C₃ | internal standard | ISO SCIENCE | 400 |
| Dehydroepiandrosterone | DHEA | standard | Sigma-Aldrich | |
| Dehydroepiandrosterone-¹³C₃ | DHEA-¹³C₃ | internal standard | in-house synthesis | 200 |
| Androstenedione | A-dione | standard | Sigma-Aldrich | |
| Androstenedione-¹³C₃ | A-dione-¹³C₃ | internal standard | in-house synthesis | 400 |
| Corticosterone | B | standard | Sigma-Aldrich | |
| Corticosterone-d₄ | B-d₄ | internal standard | ISO SCIENCE | 500 |
| Cortisol | F | standard | NIST | |
| Cortisol-d₄ | F-d₄ | internal standard I | CDN ISOTOPE | 1000 |
| Cortisone | E | standard | Sigma-Aldrich | |
| Cortisone-¹³C₃ | E-¹³C₃ | internal standard | ISO SCIENCE | 500 |

### (5) Purification of Steroid Hormones

To the residue obtained, 0.5 mL of methanol and 1 mL of water were sequentially added to get it dissolved which was applied into the OASIS MAX cartridge, a mixed-mode type solid-phase extraction (SPE) column. After the SPE column was washed with 1 mL of water, 1 mL of 40% methanol solution containing 1% acetic acid, and 1 mL of 1% pyridine solution, the steroid fraction was then eluted with 1 mL of methanol and evaporated to dryness.

### (6) Picolinate Derivatization of Steroid Hormones

The residue (containing the steroid hormones) obtained by the column purification was dried under reduced pressure for 15 minutes or longer and treated with 50 µL the reagent mixture (80 mg of 2-methyl-6-nitrobenzoic anhydride, 40 mg of picolinic acid, and 20 mg of dimethylaminopyridine in 1 mL of acetonitrile) and 10 µL of trimethylamine, and then left at room temperature for 30 minutes. After derivatization, the reaction mixture was quenched with 0.5 mL of hexane/ethyl acetate/acetic acid mixed solution (35:15:1) and purified with a normal-phase SPE column (HyperSep SI). After the SPE column was washed with 1 mL of hexane and 2 mL of hexane/ethyl acetate mixed solution (7:3), the fraction containing the derivatized steroids was eluted with 2.5 mL of acetone/hexane mixed solution (7:3), and then evaporated to dryness with a centrifugal evaporator.

### [Table 2]

**Table 2 Reagents and columns used in Example 1**

| Name | Grade | Manufacturer |
|---|---|---|
| Acetonitrile | special grade | NACALAI TESQUE, INC. |
| 2-Propanol | special grade | NACALAI TESQUE, INC. |
| Methanol | special grade | NACALAI TESQUE, INC. |
| Hexane | special grade | FUJIFILM Wako Pure Chemical Corporation |
| Triethylamine | special grade | FUJIFILM Wako Pure Chemical Corporation |
| 2-Methyl-6-nitrobenzoic anhydride | EP | Tokyo Chemical Industry Co., Ltd. |
| Dimethylaminopyridine | EP | Tokyo Chemical Industry Co., Ltd. |
| Picolinic acid | - | Tokyo Chemical Industry Co., Ltd. |
| Acetic acid | special grade | FUJIFILM Wako Pure Chemical Corporation |
| Ethyl acetate | special grade | NACALAI TESQUE, INC. |
| Trifluoroacetic acid | Wako special grade | FUJIFILM Wako Pure Chemical Corporation |
| Methyl tert-Butyl Ether | SAJ special grade | Sigma-Aldrich |
| Mix-mode solid-phase column (OASIS MAX) | - | Waters |
| Normal-phase solid-phase column (HyperSep SI) | - | Thermo Fisher Scientific |

### (7) Measurement of Amounts of Steroid Hormones

The residue was dissolved in 100 µL of 40% acetonitrile solution and the resultant was subjected to one-dimensional LC-MS/MS (see Tables 3 to 5) to measure the amounts of the steroid hormones in the hair sample.

Calculation of peak areas, calculation of peak area ratios, preparation of calibration curves, and quantification in calculation of concentrations were performed with the LC-MS/MS quantification software Analyst ver.1.6. The target compounds and their internal standards were identified on the basis of retention time and mass units of detected ions in MRM chromatograms.

For preparing calibration curves, the equation **Y** = **aX** + **b** with **1/X** weighting was used, where **X** were the concentrations of target compound (seven steroid hormones) and **Y** were the ratios of the compound peak area to the internal standard peak area. The slope **a** and the Y-intercept **b** were determined by the least-square method from the curve. The concentrations of the compounds (pg/tube) were calculated with the calibration curves and peak area ratios obtained (Table 6). Thereafter, using Excel 2016, the concentrations of the compounds (pg/tube) were divided by the amount of hair per tube (mg) to obtain the compound concentrations in hair (ng/g). Figure 11 to Figure 15 show the results.

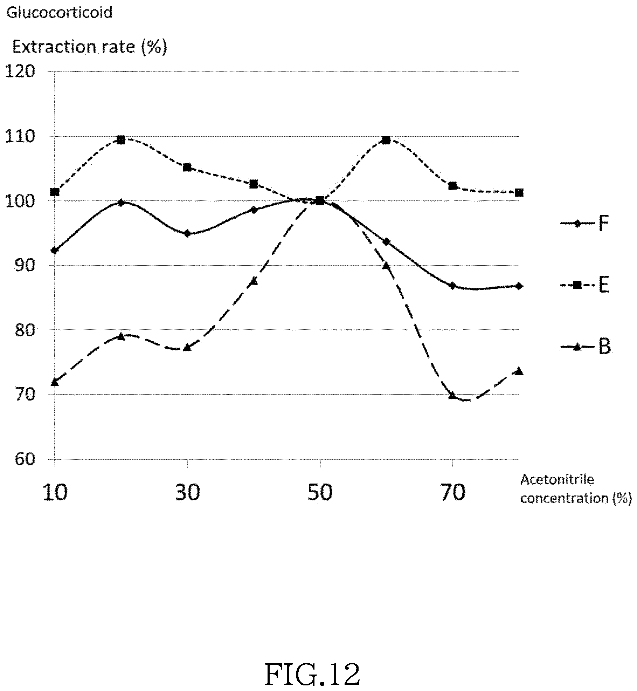

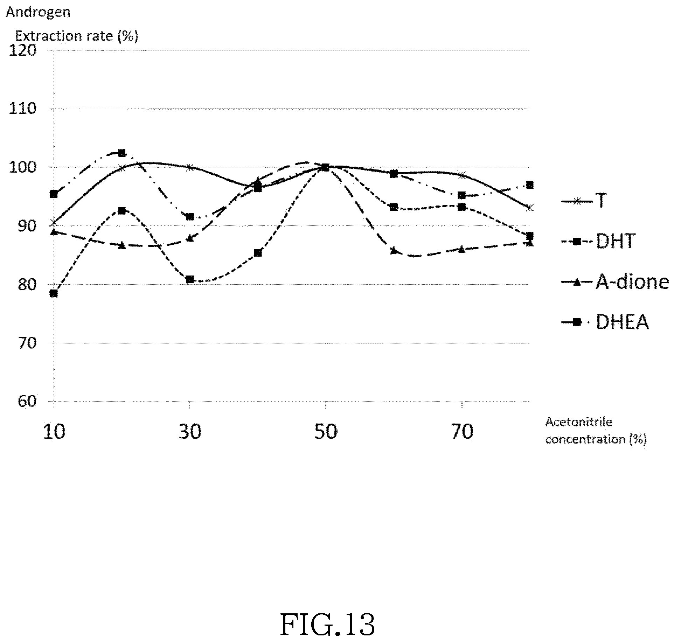

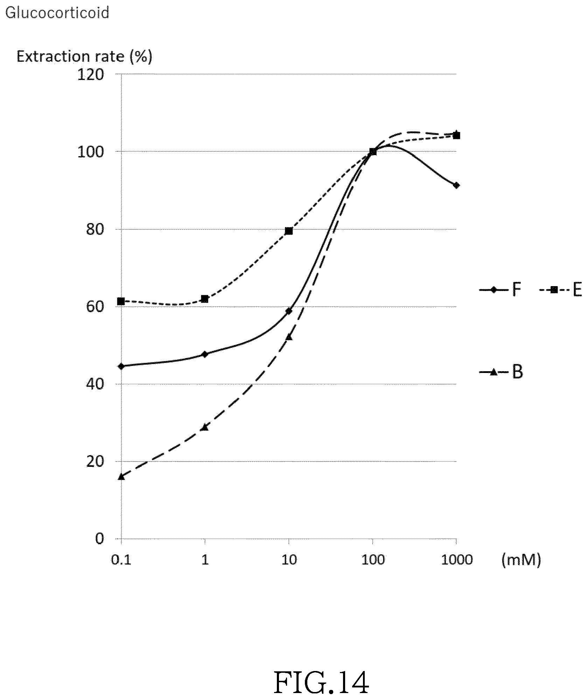

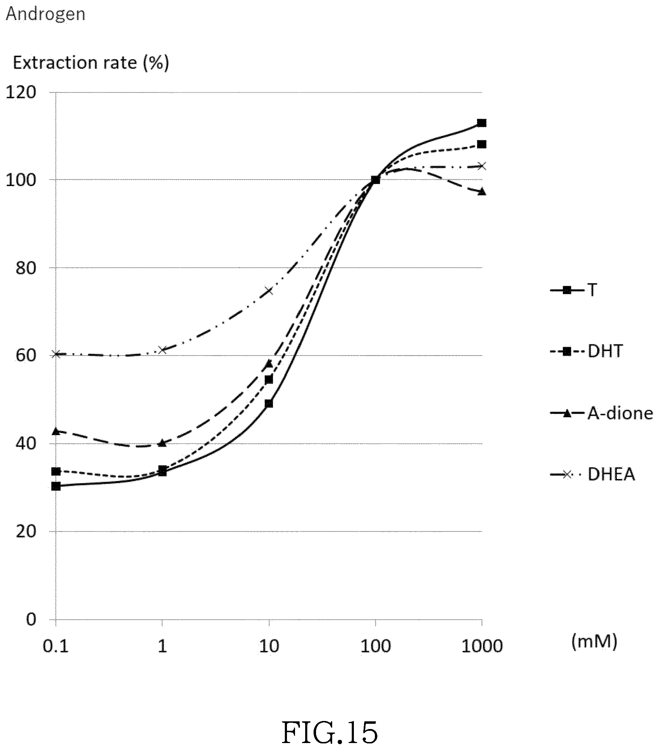

As is clear from Figure 12 and Figure 13, the extraction solvent with an acetonitrile concentration of 50 vol% exhibited good extraction rates for the seven types of steroid hormones. As is clear from Figure 14 and Figure 15, the extraction rates for the seven types of steroid hormones almost reached the plateau at the TFA concentration of 100 mM in the case of the 50 vol% aqueous acetonitrile solution.

### [Table 3]

**Table 3 Apparatuses used in Example 1**

| Name of apparatus | | Model | Supplier |
|---|---|---|---|
| LC-MS/MS system | MS/MS | API5000 | AB Sciex |
| | UHPLC | Agilent1290 Infinity LC system | Agilent Technologies |
| Analytical column | | Meteoric Core | YMC |

### [Table 4]

**Table 4 Conditions for one-dimensional LC used in Example 1**

| | |
|---|---|
| Column | Meteoric Core C18, 2.7 µm, 2.1 mm×150 mm |
| Mobile phase | gradient analysis with 0.1% formic acid solution and acetonitrile |
| Flow rate of mobile phase | 0.5 mL/min |
| Column temperature | 50 ° C |

### [Table 5]

**Table 5 Conditions for MS/MS used in Example 1**

| Compounds | Derivatization | Ion measured in one-dimensional LC-MS/MS (m/z) | | |
|---|---|---|---|---|
| | | Precursor | Product | |
| T | picolinic acid | 394.2 | 147.2 | 253.2 |
| T-¹³C₃ | | 397.2 | 150.2 | 256.2 |
| DHT | | 396.3 | 255.1 | 203.2 |
| DHT-¹³C₃ | | 399.3 | 258.1 | |
| DHEA | | 394.2 | 253.0 | 175.1 |
| DHEA-¹³C₃ | | 397.2 | 256.2 | |
| B | | 452.3 | 311.2 | 293.2 |
| B-d₄ | | 456.3 | 315.2 | |
| A-dione | | 287.2 | 109.0 | 97.0 |
| A-dione-¹³C₃ | | 290.3 | 100.2 | |
| F | picolinic acid | 468.3 | 309.3 | 450.2 |
| F-d₄ | | 472.2 | 454.1 | |
| E | | 466.3 | 448.3 | |
| E-^{l3}C₃ | | 469.3 | 451.3 | 268.3 |

**[Table 6]**

| Data for calibration curves and quantification ranges | | | | |
|---|---|---|---|---|
| Compounds | Regression equation of calibration curve (weight: 1/X) | Correlation coefficient | Quantification range (pg/tube) | Retention time (min) |
| Cortisol (F) | y= 0.528 x + 0.0014200 | r=0.9995 | 10 ~ 10000 | 2.16 |
| Cortisone (E) | y= 0.188 x + 0.0011 | r=0.9991 | 10 ~ 10000 | 2.22 |
| Corticosterone (B) | y= 2.52 x + 0.00296 | r=0.9986 | 2 ~ 2000 | 2.53 |
| Androstenedione (A-dione) | y= 1.11 x + 0.00185 | r=0.9990 | 2 ~ 2000 | 2.64 |
| Testosterone (T) | y= 2.04 x + 0.00405 | r=0.9996 | 1 ~ 1000 | 3.54 |
| Dihydrotestosterone (DHT) | y= 1.23 x + 0.00075 | r=0.9995 | 1 ~ 1000 | 4.23 |
| Dehydroepiandrosterone (DHEA) | y= 0.122 x + 0.00126 | r=0.9984 | 2 ~ 2000 | 4.11 |

### (Example 2) Examination on Extraction Solvent and Extraction Time

To examine the influence of the type of extraction solvent and duration of extraction on extraction rates for steroid hormones, different extraction solvents were used to extract steroid hormones from a human hair sample with different duration of extraction.

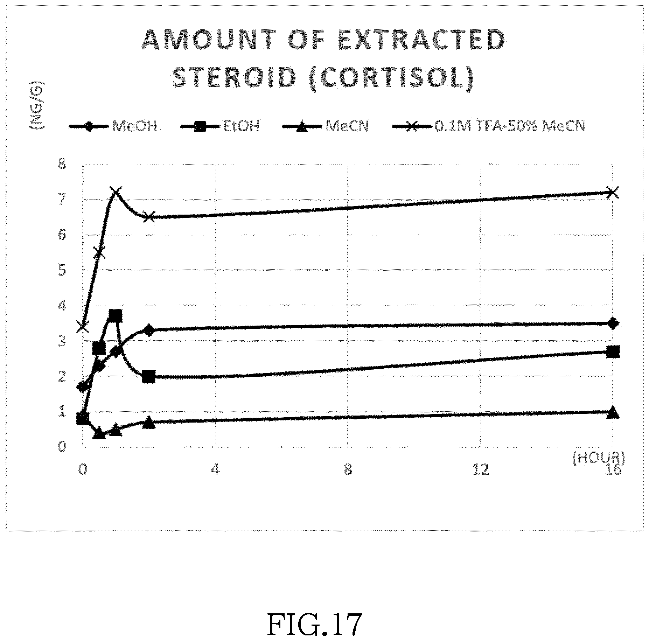

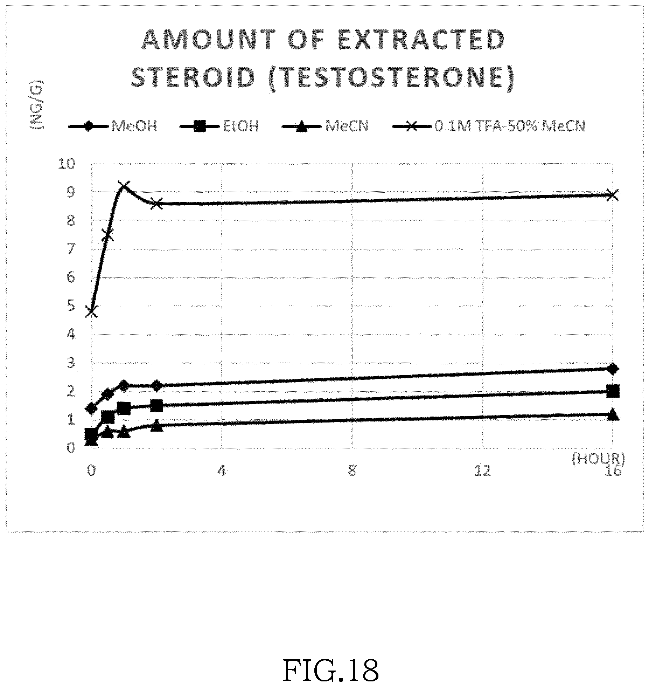

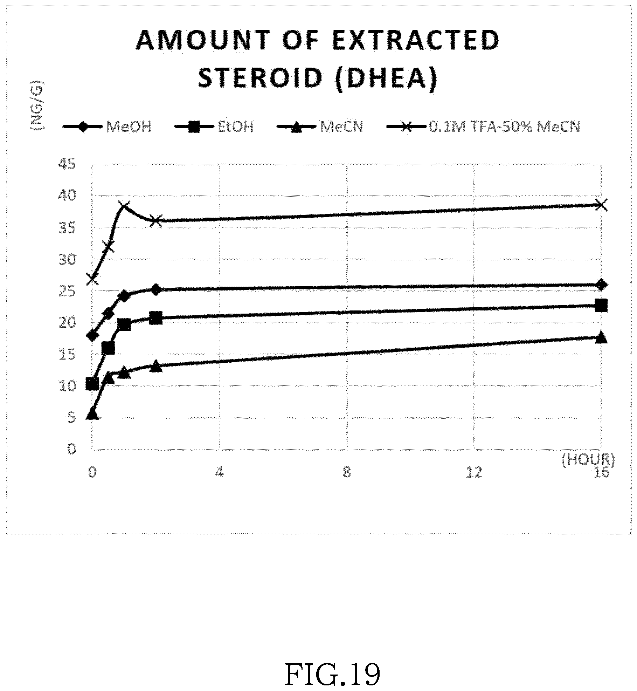

The amounts of seven types of steroid hormones were measured with the human hair sample in the same manner as in Example 1; except that methanol, ethanol, acetonitrile, and a 50 vol% aqueous acetonitrile solution containing 0.1 M trifluoroacetic acid were used as the extraction solvents, and duration of extraction was set to 0 minute, 30 minutes, 60 minute, 120 minutes, and 16 hours. Figure 16 to Figure 19 show the results.

### (Example 3) Analysis of Steroid Hormones with Small Amount of Human Hair Sample

By using a 50 vol% aqueous acetonitrile solution containing 0.1 M trifluoroacetic acid, steroid hormones were extracted from a hair sample prepared from 10 strands of hair, and the amounts of the steroid hormones were measured by two-dimensional LC-MS/MS.

### (1) Collection and Washing of Hair

Hair samples were collected from 10 healthy male volunteers and 10 hair strands in the posterior vertex of them were cut as close to the scalp as possible with scissors. The collected hair strands were cut at a length of 3-centimeter from the hair-root side and used for the measurement. The sample of 10 strands grouped by subjects was weighed with an electronic scale and washed twice with 0.5 mL 2-propanol.

### (2) Extraction, Purification, and Picolinate Derivatization of Steroid Hormones

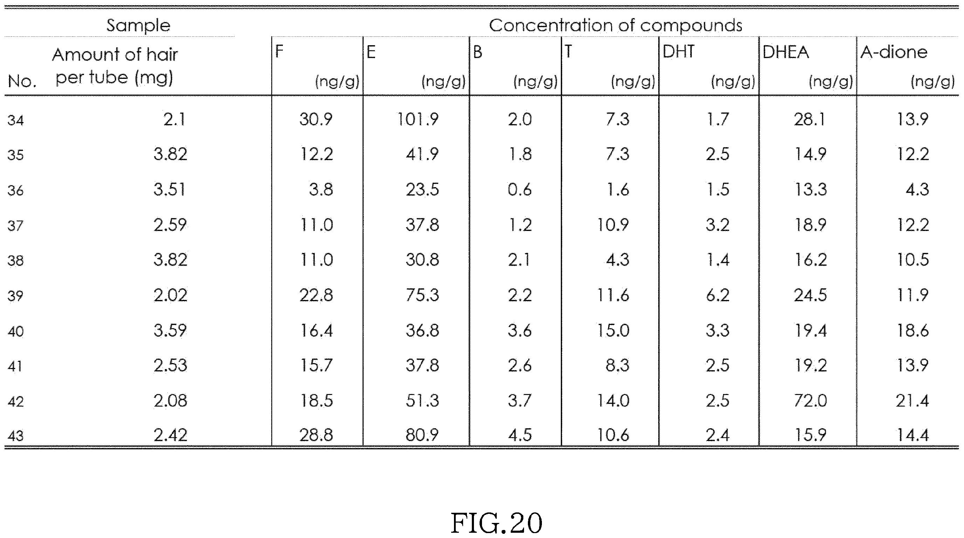

The harvested each hair samples after washing were further crushed into powder in the same manner as in Example 1, and used as the hair sample of each subject. Each of the hair samples was put in a glass spitz tube for the corresponding subject (the amounts of the hair samples from the subjects are shown in Figure 20); with the same methods as in Example 1, the steroid hormones were extracted from each of the samples by using a 50 vol% aqueous acetonitrile solution containing 0.1 M trifluoroacetic acid, and subjected to column purification followed by picolinate derivatization.

### (3) Measurement of Amounts of Steroid Hormones

Each of the residues was dissolved in 100 µL of 40% acetonitrile solution, and subjected to two-dimensional LC-MS/MS (see Tables 7 to 9) to measure the amounts of the steroid hormones in each of the hair samples. Figure 20 shows the results.

### [Table 7]

**Table 7 Apparatuses used in Example 3**

| Name of apparatus | | Model | Supplier |
|---|---|---|---|
| LC-MS/MS system | MS/MS | API5000 | AB Sciex |
| | UHPLC | Agilent1290 Infinity LC system | Agilent Technologies |
| Analytical column | | CAPCELL CORE | OSAKA SODA CO., LTD. |
| | | CAPCELL PAK | |
| | | Cortecs | Waters |

### [Table 8]

**Table 8 Conditions for two-dimensional LC used in Example 3**

| | | |
|---|---|---|
| T, DHT, DHEA, A-dione, B | Analysis mode | 2D-LC |
| | Column | 1st: CAPCELL CORE ADME, 2.7 pm, 2.1 mm×150 mm |
| | | 2nd: Cortecs C18, 1.6 µm, 2.1 mm× 150 mm |
| | Mobile phase | 1st: gradient analysis with 0.1% formic acid solution and acetonitrile |
| | | 2nd: gradient analysis with 1 mM acetic acid solution and acetonitrile |
| | Flow rate of mobile phase | 1st: 0.55 mL/min |
| | | 2nd: 0.5 mL/min |
| | Column temperature | 50°C |
| F, E | Analysis mode | 2D-LC |
| | Column | 1st: CAPCELL PAK ADME, 2 pm, 2.1 mm ×100 mm |
| | | 2nd: CAPCELL CORE C18, 2.7 pm, 3 mm× 150 mm |
| | Mobile phase | 1st: gradient analysis with 0.1% formic acid solution and acetonitrile/methanol (1:1) mixed solution |
| | | 2nd: gradient analysis with 0.1% formic acid solution and methanol |
| | Flow rate of mobile phase | 1st: 0.45 mL/min |
| | | 2nd: 0.55 mL/min |
| | Column temperature | 40°C |

### [Table 9]

**Table 9 Conditions for MS/MS used in Example 3**

| Compounds | Derivatization | Ion measured in two-dimensional LC-MS/MS (m/z) | | |
|---|---|---|---|---|
| | | Precursor | Product | |
| T | picolinic acid | 394.2 | 147.2 | 253.2 |
| T-¹³C₃ | | 397.2 | 150.2 | 256.2 |
| DHT | | 396.3 | 255.1 | 203.2 |
| DHT-¹³C₃ | | 399.3 | 258.1 | |
| DHEA | | 394.2 | 253.0 | 175.1 |
| DHEA-¹³C₃ | | 397.2 | 256.2 | |
| B | | 452.3 | 311.2 | 293.2 |
| B-d₄ | | 456.3 | 315.2 | |
| A -dione | - | 287.2 | 109.0 | 97.0 |
| A-dione-¹³C₃ | | 290.3 | 100.2 | |
| F | picolinic acid | 468.3 | 309.3 | 450.2 |
| F-d₄ | | 472.2 | 454.1 | |
| E | | 466.3 | 448.3 | |
| E-¹³C₃ | | 469.3 | 451.3 | 268.3 |

### [Table 10]

**Table 10 Data for calibration curves and quantification ranges**

| Compounds | Regression equation of calibration curve (weight: 1/X) | Correlation coefficient | Quantification range (pg/tube) | Retention time (min) |
|---|---|---|---|---|
| Cortisol (F) | y= 1.24 x + 0.0000789 | r=1.0000 | 1 ~ 5000 | 5.28 |
| Cortisone (E) | y= 0.394 x + 0.000718 | r=1.0000 | 1 ~ 5000 | 5.18 |
| Corticosterone (B) | y= 5.94 x - 0.00497 | r=0.9996 | 2 ~ 1000 | 2.55 |
| Androstenedione (A-dione) | y= 1.35 x + 0.00289 | r=0.9996 | 2 ~ 1000 | 3.00 |
| Testosterone (T) | y= 5.01 x + 0.000882 | r=0.9998 | 0.5 ~ 500 | 4.52 |
| Dihydrotestosterone (DHT) | y= 1.35 x + 0.000828 | r=0.9990 | 0.25 ~ 125 | 8.66 |
| Dehydroepiandrosterone (DHEA) | y= 0.256 x + 0.0014 | r=1.0000 | 2 ~ 1000 | 8.52 |

### (Example 4) Examination on Concentration Distributions of Steroid Hormones

To examine the concentration distributions of steroid hormones in the head, the concentrations of steroid hormones in hair samples collected from different sites of the head were measured. Two types of steroid hormones, dihydrotestosterone and cortisol, were measured.

### (1) Sites for Hair Collection

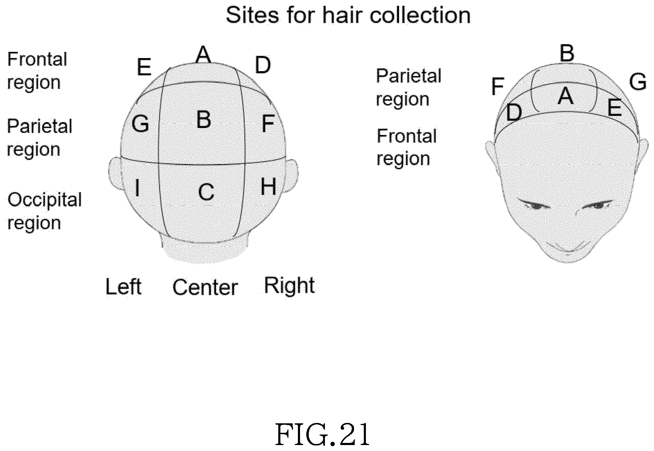

The head with hair was divided into nine regions such that **A** represents the center of the frontal region; **E** and **D** represent the left and right of the frontal region, respectively; **B** represents the center of the parietal region; **G** and **F** represent the left and right of the parietal region, respectively; **C** represents the center of the occipital region; and **I** and **H** represent the left and right of the occipital region, respectively (see Figure 21).

### (2) Collection and Washing of Hair

From each of the 9 points, **A** to **I** in Figure 21, for all eight male volunteers as subjects, 10 hair strands from each part of the head were collected. All the collected hair strands were cut as close to the scalp as possible with scissors, which were cut at a length of 3-centimeter from the hair-root side and used for the measurement. The sample of 10 strands grouped by subjects was weighed with an electronic scale and washed twice with 0.5 mL 2-propanol.

### (3) Pulverizing of Hair, and Extraction, Purification, and Picolinate Derivatization of Steroid Hormones

The harvested each hair samples after washing were further crushed into powder in the same manner as in Example 1, and used as the hair sample of each subject. Each of the hair samples was put in a glass spitz tube for the corresponding subject, and, with the same methods as in Example 1, the steroid hormones were extracted from each of the samples by using a 50 vol% aqueous acetonitrile solution containing 0.1 M trifluoroacetic acid, and subjected to column purification followed by picolinate derivatization.

### (4) Measurement and Analysis of Amounts of Steroid Hormones

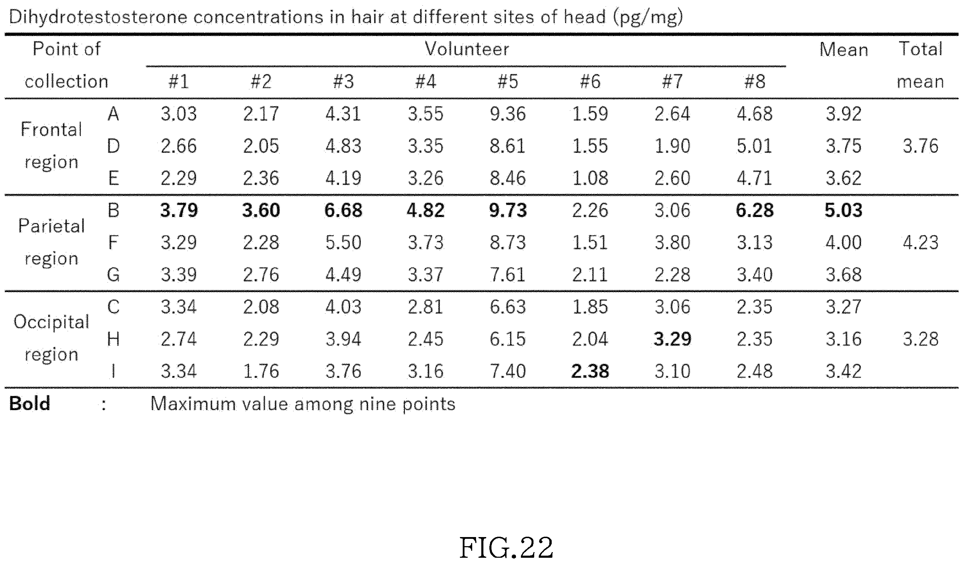

Each of the residues was dissolved in 100 µL of 40% acetonitrile solution, and subjected to two-dimensional LC-MS/MS under the same conditions as in Example 3 to measure the amounts of the steroid hormones in each of the hair samples, and Z-scores (scores obtained by normalizing every value in a dataset such that the mean of all of the values is 0 and the standard deviation is 1) were calculated. Figure 22 to Figure 24 show the results.

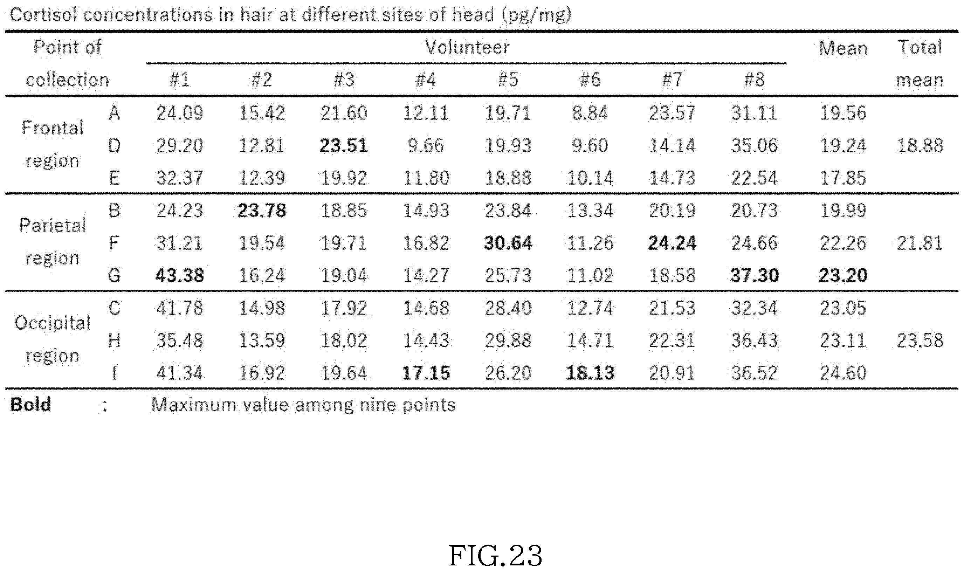

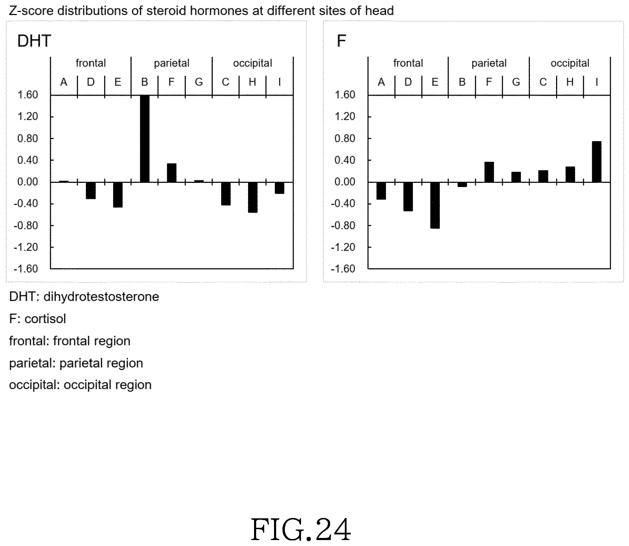

As is clear from Figure 22 and Figure 24, the hair concentration of dihydrotestosterone tended to be the highest at the center of the parietal region (B). Contrarily, there was no such trend in the region of point B when measuring the cortisol levels (Figure 23, Figure 24). It was expected that measurement of hair dihydrotestosterone concentration at the center of the parietal region can provide the method for effective evaluation and monitoring of AGA's clinical symptom, and can be utilized, for example, for preventing AGA or evaluating the effectiveness of the patient's drug therapies.

### (Example 5) Examination on Reproducibility

To examine the reproducibility of measurements of concentrations of steroid hormones in hair, three hair samples from the same region were collected from each subject, and the concentrations of steroid hormones in hair were measured. Six types of steroid hormones, cortisol, cortisone, testosterone, dihydrotestosterone, dehydroepiandrosterone, and androstenedione, were measured.

### (1) Collection, Washing, and Pulverizing of Hair

The subjects were eight male volunteers. From the center of the occipital region (C) in Figure 21 of each subject, 10 strands of hair were collected successively three times by cutting as close to the scalp as possible with scissors. The collected hair strands were cut at a length of 3-centimeter from the hair-root side and used for the measurement. The sample of 10 strands grouped by subjects was weighed with an electronic scale and washed twice with 0.5 mL 2-propanol, which was then pulverized in the same manner as in Example 1. Thus, three hair samples were obtained from each subject.

### (2) Extraction, Purification, and Picolinate Derivatization of Steroid Hormones

Each of hair samples was put in glass spitz tube, and, with the same methods as in Example 1, the steroid hormones were extracted from the hair by using a 50 vol% aqueous acetonitrile solution containing 0.1 M trifluoroacetic acid, and subjected to column purification followed by picolinate derivatization.

### (3) Measurement of Amounts of Steroid Hormones

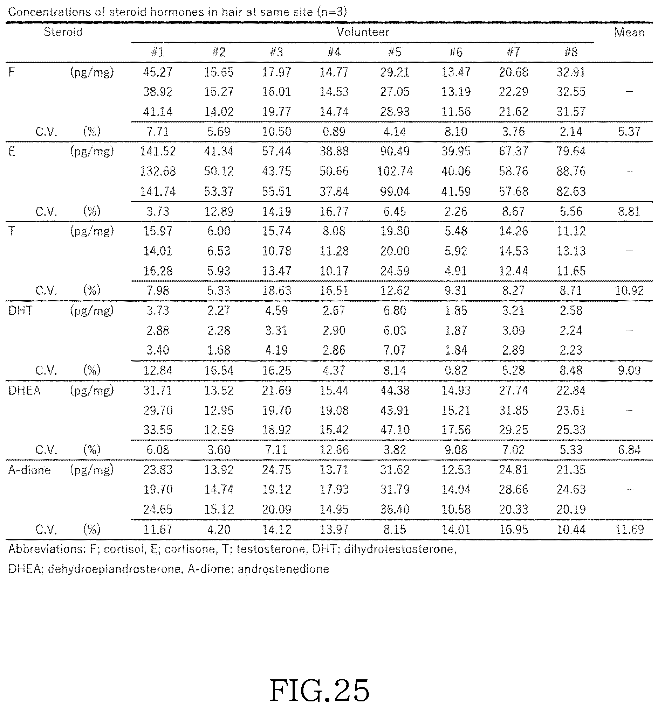

Each of the residues was dissolved in 100 µL of 40% acetonitrile solution, and subjected to two-dimensional LC-MS/MS under the same conditions as in Example 3 to measure the amounts of the steroid hormones in each of the hair samples, and C.V. values (coefficients of variation: values (%) obtained by dividing standard deviation by mean) and other values were calculated. Figure 25 shows the results.

As shown in Figure 25, the mean variability of the measured values for each steroid hormone concentration in hair ranged from 5.37 to 11.69% in terms of C.V. values (coefficients of variation). No C.V.% value exceeded 20% for all participants. These results confirmed that a reproducible measurement is possible if samples are collected from the same point.

### Industrial Applicability

The analysis method of the present invention allows analysis of a plurality of types of steroid hormones in a small amount of an animal body hair sample, thus being applicable, for example, in the fields of medicine, biochemistry, public health, stress evaluation, and AGA treatment.

### Reference Signs List

- 1: stress evaluation apparatus
- 2: hair loss cause analysis apparatus
- 3: stress evaluation apparatus
- 5: training effect measurement apparatus
- 10: analysis means
- 11: storage unit
- 12: evaluation unit
- 13: output unit
- 20: analysis means
- 21: cause analysis unit
- 22: output unit
- 30: analysis means
- 31: input unit
- 32: analysis result integration unit
- 33: output unit
- 50: analysis means
- 51: input unit
- 52: analysis result integration unit
- 53: output unit

## Claims

1. An analysis method for analyzing steroid hormones contained in a body hair sample from an animal, wherein
body hair collected from an animal is used as the body hair sample, and the analysis method comprises:
an extraction step of extracting a plurality of types of steroid hormones from the body hair sample by using a 45 vol% to 55 vol% aqueous acetonitrile solution containing 0.1 M trifluoroacetic acid; and
a measurement step of measuring the amounts of the plurality of types of steroid hormones extracted.

2. The analysis method according to claim 1, comprising a pulverizing step of pulverizing the body hair sample before the extraction step.

3. The analysis method according to claim 1 or 2, wherein, in the measurement step, the amounts of the plurality of types of steroid hormones are measured by using liquid chromatography, gas chromatography, electrophoresis, radioimmunoassay, enzyme immunoassay, mass spectrometry, or a combination of two or more thereof.

4. The analysis method according to any one of claims 1 to 3, wherein the amount of the body hair sample is 0.35 mg or more.

5. The analysis method according to any one of claims 1 to 3, wherein the amount of the body hair sample is 0.35 mg to 3.5 mg.

6. The analysis method according to any one of claims 1 to 3, wherein the amount of the body hair collected from an animal is two or more strands of body hair.

7. The analysis method according to any one of claims 1 to 3, wherein the amount of the body hair collected from an animal is 2 to 20 strands of body hair.

8. The analysis method according to any one of claims 1 to 7, wherein the plurality of types of steroid hormones are three or more types of steroid hormones.

9. The analysis method according to any one of claims 1 to 8, wherein the plurality of types of steroid hormones are selected from the group consisting of a glucocorticoid, a sex hormone, and a combination thereof.

10. The analysis method according to any one of claims 1 to 9, wherein the body hair sample is body hair collected from a human.

11. A method for stress evaluation, comprising:
a step of obtaining 2 to 20 strands of hair of a subject from the root of the hair;
an extraction step of extracting at least two types of steroid hormones selected from cortisol, cortisone, and DHEA from the hair by using a 45 vol% to 55 vol% aqueous acetonitrile solution containing 0.1 M trifluoroacetic acid;
a measurement step of measuring the amounts of the at least two types of steroid hormones extracted; and
a step of reading out measurement results for a plurality of control subjects from a storage unit and evaluating stress of the subject by comparison with the measurement results read out.

12. A method for analyzing a cause of hair loss, comprising:
a step of obtaining 2 to 20 strands of hair of a subject from the root of the hair;
an extraction step of extracting a first steroid hormone being at least one selected from cortisol, cortisone, and DHEA, and a second steroid hormone being at least one selected from DHT and testosterone, from the hair by using a 45 vol% to 55 vol% aqueous acetonitrile solution containing 0.1 M trifluoroacetic acid;
a measurement step of measuring the amounts of the first steroid hormone extracted and the second steroid hormone extracted; and
a step of analyzing a cause of hair loss by comparing the amounts of the first steroid hormone with those of the second steroid hormone.
